(19) Europäisches Patentamt
European Patent Office
Office européen des brevets

(11) Veröffentlichungsnummer : **0 166 490**
**B1**

(12) # EUROPÄISCHE PATENTSCHRIFT

(45) Veröffentlichungstag der Patentschrift : **28.09.88**

(21) Anmeldenummer : **85201008.1**

(22) Anmeldetag : **12.06.85**

(51) Int. Cl.⁴ : **C 07 C 45/71**, C 07 C 49/04, C 07 C 49/76, C 07 C 49/395, C 07 C 49/403, C 07 C 49/413, C 07 D213/50

(54) **Verfahren zur Herstellung von alpha-methylsubstituierten Ketonen.**

(30) Priorität : **15.06.84 DE 3422282**

(43) Veröffentlichungstag der Anmeldung : **02.01.86 Patentblatt 86/01**

(45) Bekanntmachung des Hinweises auf die Patenterteilung : **28.09.88 Patentblatt 88/39**

(84) Benannte Vertragsstaaten : **CH DE FR GB IT LI NL**

(56) Entgegenhaltungen :
**DE-A- 2 148 610**
**FR-A- 741 385**
**FR-A- 1 071 243**
**FR-A- 2 207 897**

(73) Patentinhaber : **BASF Aktiengesellschaft**
**Carl-Bosch-Strasse 38**
**D-6700 Ludwigshafen (DE)**

(72) Erfinder : **Lenz, Hans-Heinrich, Dr.**
**Kolpingstrasse 15**
**D-6800 Mannheim 1 (DE)**

**EP 0 166 490 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein neues Verfahren zur Herstellung von α-methylsubstituierten Ketonen durch Umsetzung der entsprechend unsubstituierten Ketone mit Methanol in der Gasphase in Gegenwart eines Metalloxids.

Es ist bekannt, höhere Ketone aus den jeweils niedrigeren Homologen aufzubauen. Im Falle der Kettenverlängerung um ein Kohlenstoffatom (Methylierung) kann man die als Ausgangsprodukt verwendeten Ketone in einer Aldolreaktion mit Formaldehyd umsetzen, aus den entstandenen Additionsprodukten Wasser abspalten und die resultierenden α-β-ungesättigten Ketone einer Hydrierreaktion an der C-C-Doppelbindung unterwerfen.

Eine andere Möglichkeit besteht beispielsweise darin, daß man die jeweils umzusetzenden Ketone in ihre Enolate überführt und diese mit Methylierungsreagenzien, wie Methylhalogenid oder Dimethylsulfat methyliert (vgl. Houben-Weyl, « Methoden der organischen Chemie », 4. Auflage, Band 7/2b, S. 1385 ff.).

Die Nachteile der genannten Verfahren liegen in der Verwendung von Hilfsbasen, die zudem teilweise sehr kostspielig sind, sowie in der Verwendung von stark toxischen Substanzen (z. B. Dimethylsulfat). Weiterhin handelt es sich bei diesen Verfahren größtenteils um mehrstufige Prozesse, bei denen die Wertprodukte oft nur in schlechten Ausbeuten und geringer Selektivität entstehen.

Aus J. Chem. Soc., Chem. Commun. 1984, 39 ist die Umsetzung von Aceton mit Methanol in der Gasphase an einem Eisen-Trägerkatalysator, dessen Trägermaterial aus Magnesiumoxid besteht, bekannt. Dabei wirkt Methanol als Vinylierungsagens, d. h. bei den dort beschriebenen Umsetzungen erhält man überwiegend Methylvinylketon als Hauptprodukt. Als Nebenprodukte entstehen neben Methylethylketon nicht näher bezeichnete gesättigte und ungesättigte Ketone die 5 Kohlenstoffatome aufweisen, sowie größere Mengen an Isopropanol, das vermutlich durch einen Wasserstofftransfer von Methanol zu Aceton gebildet wird.

Nach dieser Lehre ist zu erwarten, daß auch andere Ketone, die in α-Position Wasserstoffatome besitzen, mit Methanol zu den entsprechenden Vinylketonen reagieren.

Demgegenüber wurde nun überraschenderweise gefunden, daß Methanol nicht als Vinylierungsagens wirkt, sondern, daß man α-methylsubstituierte Ketone der allgemeinen Formel

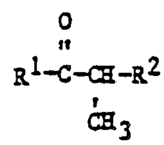

in der $R^1$ einen unsubstituierten oder gegebenenfalls durch niedere Alkylreste substituierten Aryl- oder Heteroarylrest oder einen tert-Alkylrest und $R^2$ ein Wasserstoffatom oder einen geradkettigen oder verzweigten Alkylrest mit 1 bis 12 Kohlenstoffatomen bedeuten, oder $R^1$ und $R^2$ zusammen eine unsubstituierte oder gegebenenfalls durch niedere Alkylreste substituierte Alkylengruppe $-(CH_2)_n-$ bedeuten, worin n eine ganze Zahl von 3 bis 13 darstellt, vorteilhaft erhält, wenn man die entsprechend unsubstituierten Ketone, die mindestens zwei geminale Wasserstoffatome in α-Position aufweisen müssen, mit Methanol in der Gasphase bei einer Temperatur von 350 bis 500 °C und einen Druck von 1 bis 20 bar in Gegenwart eines Metalloxids, ausgewählt aus der Gruppe bestehend aus den Oxiden der Metalle Cer, Chrom, Eisen, Magnesium und Mangan, umsetzt.

Die Reaktionsprodukte stellen α-methylsubstituierte Ketone der oben genannten allgemeinen Formel dar, in der $R^1$ einen unsubstituierten oder gegebenenfalls durch niedere Alkylreste substituierten Aryl- oder Heteroarylrest oder einen tert-Alkylrest und $R^2$ ein Wasserstoffatom oder einen geradkettigen oder verzweigten Alkylrest mit 1 bis 12 Kohlenstoffatomen bedeuten, oder $R^1$ und $R^2$ zusammen eine unsubstituierte oder gegebenenfalls durch niedere Alkylreste substituierte Alkylengruppe $-(CH_2)_n-$ bedeuten, worin n eine ganze Zahl von 3 bis 13 darstellt.

Als Aryl- bzw. Heteroarylreste sind beispielsweise der Phenyl-, Naphthyl- bzw. Pyridyl-, Pyrimidyl-, Pyrrolyl-, Furyl- oder Thienylrest zu nennen. Diese Reste können in unsubstituierter oder gegebenenfalls durch niedere Alkylreste substituierter Form vorliegen, wobei als niedere Alkylreste, z. B. dser Methyl-, Ethyl-, Isopropyl- oder Butylrest in Betracht kommen.

Als tert-Alkylreste sind beispielsweise der tert-Butyl-, tert-Pentyl- oder 1,1-Dimethylbutylrest zu nennen.

Vorzugsweise bedeutet $R^1$ einen unsubstituierten oder mit einer niederen Alkylgruppe substituierten Phenyl- oder Pyridylrest sowie einen tert-Alkylrest.

Als Beispiele für $R^2$ sind neben dem Wasserstoffatom der Methyl-, Ethyl-, Propyl-, Isopropyl, Butyl-, sec-Butyl oder tert-Butylrest zu nennen.

Wenn $R^1$ und $R^2$ zusammen einen Alkylenrest $-(CH_2)_n-$ bilden, steht n für eine ganze Zahl von 3 bis 13, bevorzugt für 3, 5, 6 oder 10. Der Alkylenrest kann unsubstituiert oder durch niedere Alkylreste substituiert sein, wobei als niedere Alkylreste, z. B. $C_1$-$C_6$-Alkylreste, beispielsweise Methyl-, Ethyl-, Isopropyl- oder Butylreste in Betracht kommen. Es kann eine Mono-, Di- oder Polysubstitution vorliegen.

Eine besondere Art der Substitution des Alkylenrests besteht in einer Verbrückung, beispielsweise unter Bildung eines bicyclischen Systems.

Als Beispiele für Alkylenreste $—(CH_2)_n—$ sind insbesondere zur nennen der Propylen-, Pentylen-, Hexylen- und Decylenrest.

Als Ausgangsprodukte dienen die entsprechend unsubstituierten Ketone, die in α-Position mindestens zwei geminale (d. h. an das selbe Kohlenstoffatom gebundene) Wasserstoffatome aufweisen müssen.

Für den Fall, daß zwei geminale Wasserstoffatome in der α-Position vorhanden sind, wird im erfindungsgemäßen Verfahren eine Methylgruppe in dieser Position eingebaut (Formel: $R^2$ = Alkyl). Eine Weitermethylierung wird nicht beobachtet.

Für den Fall, daß die als Ausgangsprodukt zu verwendenden Ketone drei geminale Wasserstoffatome in α-Position aufweisen (Methylketone), ist entweder der gezielte Einbau einer Methylgruppe (Formel: $R^2$ = H) oder auch zweier Methylgruppen (Formel: $R^2$ = Methyl) in dieser Position möglich.

Üblicherweise erhält man in diesem Falle zunächst gezielt das Monomethylprodukt. Durch geeignete Wahl der Reaktionsbedingungen, z. B. höhere Verweilzeit im Reaktor oder Erhöhung der Reaktionstemperatur kann man aber auch direkt zum Dimethylprodukt gelangen.

Im Falle von cyclischen Ausgangsketonen kann die Methylierung in α- und α,α'-Stellung erfolgen.

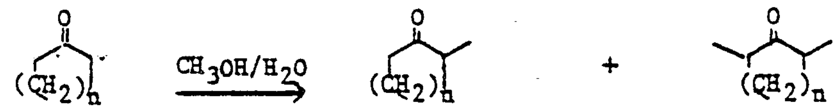


Cyclopentanon, Cycloheptanon, Cyclooctanon und Cyclododecanon lassen sich mit guten Selektivitäten in -oder α,α'-Stellung methylieren bzw. bismethylieren. Das Verhältnis der beiden Produkte ist durch Änderung der Temperatur und Verweilzeit variierbar. Durch Zurückführen des Monomethylderivates läßt sich die Selektivität vollkommen in Richtung des α,α'-Dimethylcycloalkanons verschieben. Eine hohe Selektivität an dem Monomethylderivat kann man durch niedrigen Umsatz des Ausgangsmaterials erreichen (s. u.) Wichtigste Nebenprodukte sind vermutlich durch Wasserstoffübertragung vom Methanol auf das Keton entstehende Cycloalkanole und α,β-ungesättigte Ketone. Dies erklärt auch die niedrige Selektivität beim Einsatz von Cyclohexanon ; durch Aromatisierung entstehen Phenol, o-Kresol und 2,6-Dimethylphenol. α- bzw. β-Tetralon neigen gleichfalls zur Aromatisierung. Dagegen lassen sich geminal disubstituierte Cyclohexanone in guten Ausbeuten methylieren :

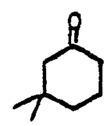

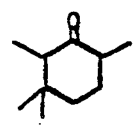

80 %

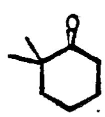

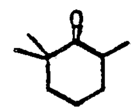

85 %

Wenn die Sechsringketone nicht geminal disubstituiert sind, besteht eine Tendenz zur Aromatisierung.

Wenn man Campher als bicyclisches Ausgangsketon methyliert, erhält man ein Gemisch aus endo- und exo-α-Methylcampher im molaren Verhältnis 3 : 1. Das zeigt die nachfolgende Gleichung :

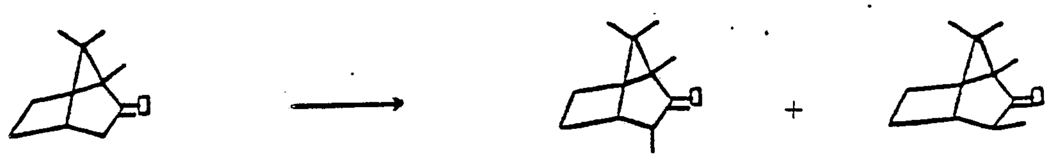

3 : 1

Die Cycloalkanole werden vorzugsweise mit einem Überschuß Methanol in Gegenwart von Wasser bei 400-480 °C, insbesondere 400-450 °C, bevorzugt an einem magnesiumoxidkatalysator, umgesetzt.

Das erfindungsgemäße Verfahren wird in Gegenwart eines Katalysators, nämlich eines Metalloxids, ausgewählt aus der Gruppe, bestehend aus den Oxiden der Metalle, Cer, Chrom, Eisen, Magnesium und

Mangan durchgeführt. Beispielsweise sind zu nennen Cer(IV)oxid, Chrom(III)oxid, Eisen(III)oxid, Magnesiumoxid oder Mangan(II)oxid. Die Verwendung von Magnesiumoxid ist bevorzugt.

Die Herstellung dieser Metalloxide ist bekannt. Es handelt sich um handelsübliche Produkte. Wie in der Katalysatortechnik üblich, werden sie vorteilhaft in geformtem Zustand, beispielsweise in Form von Tabletten, Strängen, Pillen, Kugeln oder Ringen eingesetzt.

Die Durchführung des erfindungsgemäßen Verfahrens erfolgt in der Gasphase bei einer Temperatur von 350 bis 500 °C, vorzugsweise 400 bis 450 °C und bei einem Druck von 1 bis 20 bar, vorzugsweise bei atmosphärischem Druck.

Das Molverhältnis der beiden Reaktanden Keton und Methanol beträgt 1 : 1 bis 1 : 10. Vorzugsweise arbeitet man mit einem Molverhältnis Keton : Methanol von 1 : 2 bis 1 : 4.

In manchen Fällen ist es von Vorteil, die Umsetzung in Gegenwart von Wasser vorzunehmen. Dabei empfiehlt es sich Wasser in einem Molverhältnis Wasser : Keton von 1 : 1 bis 5 : 1 zuzusetzen.

Das erfindungsgemäße Verfahren wird zweckmäßig so durchgeführt, daß man ein Gemisch der Reaktionspartner im genannten Molverhältnis, gegebenenfalls in Anwesenheit von Wasser, zunächst kontinuierlich einem Verdampfer zuführt, wo eine vollständige Verdampfung stattfindet. Die resultierende Gasmischung wird dabei auf eine Temperatur gebracht, die mit der im Reaktor benötigten Reaktionstemperatur vergleichbar ist. Zweckmäßig wählt man aber eine etwas höhere Temperatur im Verdampfer, um so eventuelle Wärmeverluste auf dem Weg zum Reaktor auszugleichen.

Das Gasgemisch tritt dann in den auf die erforderliche Reaktionstemperatur erhitzten Reaktor ein. Als Reaktor dient dabei vorteilhaft ein Rohrreaktor, in dessen Innenraum sich der Katalysator befindet.

Als typische aber nicht limitierende Katalysatorbelastung wählt man z. B. 250 bis 450 ml (bezogen auf den flüssigen Aggregatzustand) flüssiges Reaktionsgemisch je Liter Katalysator und Stunde.

Die Verweilzeit im Reaktor beträgt ca. 6 bis 15 sec. Im Fall einer « doppelten Methylierung » (vgl. oben) empfiehlt es sich, eine Verweilzeit von ca. 6 bis 20 sec. einzuhalten.

Das den Reaktor verlassende gasförmige Reaktionsgemisch wird anschließend kondensiert und kann dann durch fraktionierende Destillation gereinigt werden.

Die Vorteile des erfindungsgemäßen Verfahrens liegen im gezielten Ablauf der Reaktion, d. h. Nebenprodukte, wie die toxischen Vinylketone und deren Polymere oder die durch Wasserstofftransfer von Methanol zu Keton entstehenden Alkohole bilden sich nur in geringem Maße.

Weiterhin wird mit einem wesentlich geringeren Überschuß an Methanol gearbeitet als er im Stande der Technik üblich ist.

Schließlich ist der Umsatz an eingesetztem Keton sehr hoch (65 bis 100 %). Gleichzeitig entstehen dabei die Zielprodukte in hoher Selektivität. Dies ist überraschend, da im bekannten Verfahren (loc. cit.) mit niedrigem Umsatz (max. 37,2 %) nur schlechte Selektivitätswerte erreicht werden und üblicherweise mit steigendem Umsatz die Selektivitätswerte weiter abnehmen.

Die mittels des erfindungsgemäßen Verfahrens erhaltenen methylierten Ketone sind wertvolle Zwischenprodukte für die Synthese von Pflanzenschutzmitteln. Einige von ihnen haben auch Bedeutung als Riechstoffe.

Die nachfolgenden Beispiele sollen die Erfindung näher erläutern.

### Beispiel 1

120 ml (entsprechend 92,5 g) eines Gemisches aus 100 g 2,2-Dimethylbutan-3-on, 128 g Methanol und 18 g Wasser wurden pro Stunde kontinuierlich verdampft und über 400 ml Magnesiumoxid in Tablettenform geleitet. Das erhaltene gasförmige Reaktionsprodukt wurde kondensiert und gaschromatographisch analysiert. Nach Abzug von Methanol und Wasser wurden folgende Ergebnisse erhalten (Angabe in Gew.-%) :

| | Reaktionstemperatur | |
|---|---|---|
| | 410 °C | 425 °C |
| 2,2-Dimethylpentan-3-on | 61,5 | 62,0 |
| 2,2,4-Trimethylpentan-3-on | 3,9 | 4,1 |
| 2,2-Dimethylbutan-3-on | 24,8 | 20,9 |

### Beispiel 2

120 ml (entsprechend 103 g) eines Gemisches aus 134 g Propiophenon, 128 g Methanol und 18 g Wasser wurden pro Stunde kontinuierlich verdampft und über 400 ml Magnesiumoxid in Tablettenform geleitet. Das erhaltene gasförmige Reaktionsgemisch wurde kondensiert und gaschromatographisch analysiert. Nach Abzug von Methanol und Wasser wurden folgende Ergebnisse erhalten (Angabe in Gew.-%) :

| | Reaktionstemperatur | |
|---|---|---|
| | 420°C | 460 % |
| 1-Phenyl-2-methylpropan-1-on | 46,6 | 64,7 |
| Propiophenon | 34,1 | 15,8 |

## Beispiel 3

120 ml (entsprechend 101 g) eines Gemisches aus 121 g 3-Acetylpyridin, 320 g Methanol und 36 g Wasser wurden pro Stunde kontinuierlich verdampft und über 400 ml Magnesiumoxid in Tablettenform geleitet. Das erhaltene gasförmige Reaktionsgemisch wurde kondensiert und gaschromatographisch analysiert. Nach Abzug von Methanol und Wasser wurden folgende Ergebnisse erhalten (Angabe in Gew.-%) :

| | Reaktionstemperatur | |
|---|---|---|
| | 400°C | 420°C |
| 1-Pyrid-3-ylpropan-1-on | - | - |
| 1-Pyrid-3-yl-2-methylpropan-1-on | 67,2 | 48,0 |
| 1-Pyrid-3-yl-2-methylpropan-1-ol | 19,3 | 26,5 |
| 3-Acetylpyridin | - | - |

## Beispiele 4 bis 8

In den Beispielen 4 bis 8 wurden cyclische Ketone eingesetzt. Das jeweilige Cycloalkanon wurde mit einem Überschuß Methanol in Gegenwart von Wasser bei verschiedenen Verweilzeiten über den MgO-Kontakt gefahren. Die Temperatur lag zwischen 400 und 450 °C. In der nachfolgenden Tabelle sind Umsätze und Selektivitäten zusammengefaßt.

Tabelle 1

α-Alkylierung von Cycloalkanonen

| Ringgröße (n + 2) | Umsatz [%] | Selektivität [%] Monomethyl | Dimethyl | $\Sigma$ |
|---|---|---|---|---|
| 5 | 82 | 40 | 36 | 76 |
| 6 | 93 | 21 | 25 | 46 |
| 7 | 88 | 24 | 48 | 72 |
| 8 | 80 | 46 | 30 | 76 |
| 12 | 94 | 46 | 33 | 79 |

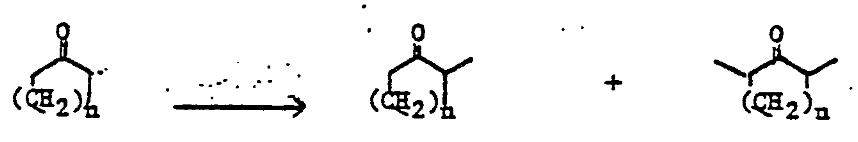

## Beispiel 9

2-Methylcyclodopentanon ist ein Vorprodukt für einen Aromastoff. Die Cyclopentanonmethylierung wurde daraufhin hinsichtlich Reaktionstemperaturen, Verweilzeit, Molverhältnis der Reaktionspartner variiert, um den Einfluß dieser Parameter auf Umsatz und Selektivität zu untersuchen. Die Ergebnisse sind in der nachstehenden Tabelle 2 zusammengefaßt.

Tabelle 2

Variation der Verfahrensparameter bei der Herstellung von 2-Methylcyclopentanon

| Temperatur [°C] | Verweilzeit [sec] | Molverhältnis $CH_3OH$:Cyclopentanon | Selektivitäten [%] Umsatz [%] | α-Methyl | α,α'-Dimethyl |
|---|---|---|---|---|---|
| 400 | 4 | 15 : 1 | 70 | 20 | 15 |
| 400 | 2 | 15 : 1 | 55 | 25 | 11 |
| 420 | 2 | 15 : 1 | 75 | 16 | 19 |
| 400 | 5 | 5 : 1 | 54 | 32 | 9 |
| 420 | 5 | 5 : 1 | 69 | 24 | 12 |
| 420 | 6 | 2 : 1 | 41 | 47 | 8 |
| 420 | 7 | 1 : 1 | 27 | 60 | 4 |
| 440 | 7 | 1 : 1 | 36 | 61 | 6 |
| 460 | 7 | 1 : 1 | 46 | 59 | 6 |

**Patentansprüche**

1. Verfahren zur Herstellung von α-methylsubstituierten Ketonen der allgemeinen Formel

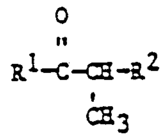

in der $R^1$ einen unsubstituierten oder gegebenenfalls durch niedere Alkylreste substituierten Aryl- oder Heteroarylrest oder einen tert-Alkylrest und $R^2$ ein Wasserstoffatom oder einen geradkettigen oder verzweigten Alkylrest mit 1 bis 12 Kohlenstoffatomen bedeuten, oder $R^1$ und $R^2$ zusammen eine unsubstituierte oder gegebenenfalls durch niedere Alkylreste substituierte Alkylengruppe $—(CH_2)_n—$ bedeuten, worin n eine ganze Zahl von 3 bis 13 darstellt, dadurch gekennzeichnet, daß man die entsprechend unsubstituierten Ketone, die mindestens zwei geminale Wasserstoffatome in α-Position aufweisen müssen, mit Methanol in der Gasphase bei einer Temperatur von 350 bis 500 °C und einem Druck von 1 bis 20 bar in Gegenwart eines Metalloxids, ausgewählt aus der Gruppe bestehend aus den Oxiden der Metalle Cer, Chrom, Eisen, Magnesium und Mangan umsetzt.

2. Verfahren gemäß Anspruch 1, dadurch gekennzeichnet, daß man die Umsetzung bei einer Temperatur von 400 bis 450 °C durchführt.

3. Verfahren gemäß Anspruch 1, dadurch gekennzeichnet, daß man die Umsetzung bei atmosphärischem Druck durchführt.

4. Verfahren gemäß Anspruch 1, dadurch gekennzeichnet, daß man die Umsetzung in Gegenwart von Magnesiumoxid durchführt.

5. Verfahren gemäß Anspruch 1, dadurch gekennzeichnet, daß man die Umsetzung mit einem Molverhältnis Keton : Methanol von 1 : 1 bis 1 : 10 durchführt.

**Claims**

1. A process for the preparation of an α-methyl-substituted ketone of the formula

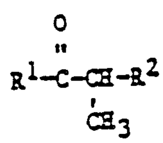

where $R^1$ is unsubstituted or optionally lower alkyl-substituted aryl or hetaryl or a tert.-alkyl radical, and $R^2$ is hydrogen or straight-chain or branched alkyl of 1 to 12 carbon atoms, or $R^1$ and $R^2$ together form an unsubstituted or optionally lower alkyl-substituted alkylene group $—(CH_2)_n—$, where n is an integer from 3 to 13, wherein the corresponding unsubstituted ketone, which must possess two or more geminal hydrogen atoms in the α-position, is reacted with methanol in the gas phase at from 350 to 500 °C and under from 1 to 20 bar in the presence of a metal oxide selected from the group consisting of the oxides of the metals cerium, chromium, iron, magnesium and manganese.

2. A process as claimed in claim 1, wherein the reaction is carried out at from 400 to 450 °C.

3. A process as claimed in claim 1, wherein the reaction is carried out under atmospheric pressure.

4. A process as claimed in claim1, wherein the reaction is carried out in the presence of magnesium oxide.

5. A process as claimed in claim 1, wherein the reaction is carried out using a molar ratio of ketone to methanol of from 1 : 1 to 1 : 10.

**Revendications**

1. Procédé de préparation de cétones substituées par méthyle en α, de formule générale :

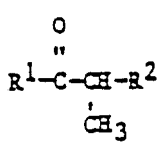

dans laquelle $R^1$ représente un reste aryle ou hétéroaryle non substitué ou éventuellement substitué par des restes alkyle inférieurs, ou un reste tert-alkyle et $R^2$ un atome d'hydrogène ou un reste alkyle de 1 à

12 atomes de carbone à chaîne droite ou ramifiée, ou $R^1$ et $R^2$ représentent ensemble un groupe alkylène $-(CH_2)_n-$ non substitué ou éventuellement substitué par des restes alkyle inférieurs, n y représentant un nombre entier de 3 à 13, caractérisé par le fait que l'on fait réagir les cétones non substituées correspondantes, qui doivent posséder en position α au moins deux atomes d'hydrogène géminaux, avec du méthanol, en phase gazeuse, à une température de 350 à 500 °C et une pression de 1 à 20 bar, en présence d'un oxyde métallique choisi dans le groupe constitué des oxydes des métaux cérium, chrome, fer, magnésium et manganèse.

2. Procédé selon la revendication 1, caractérisé par le fait que l'on effectue la réaction à une température de 400 à 450 °C.

3. Procédé selon la revendication 1, caractérisé par le fait que l'on effectue la réaction à la pression atmosphérique.

4. Procédé selon la revendication 1, caractérisé par le fait que l'on effectue la réaction en présence d'oxyde de magnésium.

5. Procédé selon la revendication 1, caractérisé par le fait que l'on effectue la réaction avec un rapport molaire, cétone/méthanol de 1/1 à 1/10.